# FASCICULE DE BREVET EUROPEEN

(11) **EP 1 982 648 B1**
(45) Date de publication et mention de la délivrance du brevet: **08.07.2015**
(21) Numéro de dépôt: 08103535.4
(22) Date de dépôt: 15.04.2008
(51) Int. Cl.: A61B 5/042, A61B 5/0408, A61B 5/0478

(54) **Electrode cutanée**
Hautelektrode
Skin electrode

(30) Priorité: 17.04.2007 FR 0754513
(43) Date de publication de la demande: 22.10.2008
(73) Titulaire: Microvitae Technologies, 13790 Peynier (FR)
(72) Inventeur: HERVE, Thierry, 38610, GIERES (FR)
(74) Mandataire: Thibon, Laurent

(56) Documents cités:
- EP-A- 0 805 489
- WO-A-97/23865
- US-A- 4 969 468
- US-A1- 2003 097 165
- US-A1- 2004 116 991
- US-A1- 2006 265 039
- US-A1- 2007 066 876
- ANONYME: "Kapton" WIKIPEDIA, THE FREE ENCYCLOPEDIA, [Online] 13 septembre 2006 (2006-09-13), XP002462633 Extrait de l'Internet: URL:http://web.archive.org/web/20060913000 000/http://en.wikipedia.org/wiki/Kapton> [extrait le 2007-12-14]

## Description

### Domaine de l'invention

La présente invention concerne une électrode cutanée, c'est-à-dire une électrode destinée à mesurer un signal électrique apparaissant à la surface de la peau d'un être vivant, humain ou animal.

### Exposé de l'art antérieur

De façon classique, de telles électrodes doivent, pour recueillir des signaux suffisants être constituées d'une plaque métallique d'un relativement grand diamètre (1 à 2 cm). Ces électrodes sont relativement épaisses et doivent être collées sur la surface de la peau en assurant une bonne adhérence, c'est-à-dire que le collage doit être relativement fort ce qui est mal adapté à des zones fragiles de la peau, ou à des peaux fragiles comme celles de prématurés.

D'autre part, ces électrodes doivent être généralement associés à un gel d'amélioration du contact entre la peau et la surface métallique. Ce gel à tendance à sécher au cours du temps, ce qui est mal adapté à des surveillances à long terme (24 à 72 heures). De plus, en raison de leurs grandes dimensions, ces électrodes sont mal adaptées à être utilisées dans des zones velues du corps (le corps d'un animal ou le crâne d'un être humain). On a donc besoin d'une électrode qui, tout en étant de petite dimension, assure un bon contact et un bon prélèvement de signaux électriques à la surface de la peau.

US 2003/0097165 A1, WO 97/23865, US 4969468, US 2004/0116991 A1 et US 2006/0265039 A1 divulguent des réseaux d'électrodes.

### Résumé de l'invention

Selon un aspect de la présente invention, il est donc prévu une électrode cutanée comprenant, sur un support souple d'une épaisseur inférieure à 10 µm, un plot d'une épaisseur inférieure à 30 µm muni de stries d'une profondeur supérieure à 10 µm, les surfaces au moins de ce plot étant conductrices et étant connectées à une zone de prise de connexion.

Selon un mode de réalisation de la présente invention, l'électrode cutanée comprend une première couche isolante support d'une épaisseur inférieure à 10 µm ; une couche métallique d'une épaisseur inférieure à 20 µm munie de stries d'une profondeur supérieure à 10 µm au niveau d'une zone de contact ; et une deuxième couche isolante d'une épaisseur inférieure à 10 µm revêtant la structure en dehors des zones de contact et d'une zone de prise de connexion.

Selon un mode de réalisation de la présente invention, les première et deuxième couches isolantes sont des couches de polyimide d'une épaisseur de l'ordre de 2 à 3 µm.

Selon un mode de réalisation de la présente invention, la couche métallique comprend une première couche métallique d'une épaisseur inférieure à 5 µm ; et une deuxième couche métallique formée sur la première couche métallique à l'emplacement de la zone de contact cutané, d'une épaisseur supérieure à 5 µm et nervurée sur toute son épaisseur, les nervures étant ménagées à un pas de l'ordre de 0,5 à 3 µm.

Selon un mode de réalisation de la présente invention, l'électrode cutanée comprend, sur la première couche isolante, une extension de la couche métallique jusqu'à une zone de connexion.

Selon un mode de réalisation de la présente invention, l'électrode cutanée a une surface de l'ordre de 0,1 cm².

La présente invention prévoit également un procédé de fabrication d'une telle électrode cutanée.

Selon un mode de réalisation de la présente invention, ce procédé de fabrication d'une électrode cutanée comprend les étapes consistant à former sur un support une couche sélectivement gravable ; former une première couche de matière plastique ; former une première couche métallique ; former une deuxième couche de matière plastique ; ouvrir dans la deuxième couche en matière plastique au-dessus de la première couche métallique des ouvertures et dans des régions respectives de contact et de connexion ; former sur la première couche métallique, dans la région de contact, des surépaisseurs métalliques nervurées d'une hauteur de 5 à 20 µm, à un pas de l'ordre de 1 à 5 µm ; et séparer du substrat l'ensemble de la première couche isolante et des éléments qu'elle supporte, par élimination de la couche sélectivement gravable.

Selon un mode de réalisation de la présente invention, le support est une plaquette de silicium et la couche sélectivement gravable est une couche d'oxyde de silicium.

Selon un mode de réalisation de la présente invention, les couches de matière plastique sont des couches de polyimide d'une épaisseur de 2 à 10 µm, de préférence de 3 à 5 µm.

Selon un mode de réalisation de la présente invention, la première couche métallique est une couche d'or d'une épaisseur d'environ 1 µm.

### Brève description des dessins

Ces objets, caractéristiques et avantages, ainsi que d'autres seront exposés en détail dans la description suivante de modes de réalisation particuliers faite à titre non-limitatif en relation avec les figures jointes parmi lesquelles:
les figures 1A et 1B représentent une vue de côté en coupe et une vue de dessus schématique d'une électrode cutanée selon un exemple de réalisation de la présente invention ;
les figures 2A à 2E sont des vues en coupe illustrant des étapes successives d'un exemple de procédé de fabrication d'une électrode cutanée selon un mode de réalisation de la présente invention.

Comme cela est habituel dans le domaine de la représentation des microdispositifs, les diverses figures ne sont pas tracées à l'échelle.

### Description détaillée

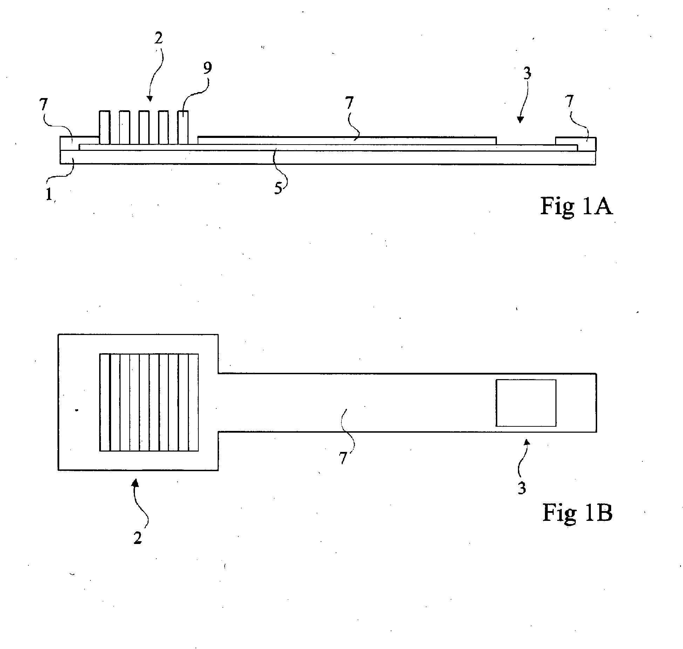

Les figures 1A et 1B représentent de façon schématique une vue de côté en coupe et une vue de dessus d'une électrode cutanée. Cette électrode comprend un support 1 en une matière plastique très mince, par exemple de 2 à 10 µm, de préférence de 3 à 5 µm. Du côté gauche des figures 1A et 1B est formée l'électrode cutanée proprement dite 2 destinée à entrer en contact avec la peau là ou l'on veut effectuer une prise de signal. Du côté droit des figures une région de connexion 3 est destinée à établir une connexion avec un conducteur dont l'autre extrémité sera connectée à un appareil de mesure, d'alarme, ou autre.

Comme le montre la vue en coupe de la figure 1A, la plus grande partie du support 1 est revêtue d'une couche conductrice mince 5, d'une épaisseur de l'ordre du µm, par exemple de 0,5 à 3 µm, de préférence de 0,5 à 1,5 µm. La couche conductrice 5, par exemple une couche d'or, est revêtue, sauf à l'emplacement de la région de contact cutanée 2 et de la région de connexion 3 d'une couche d'une matière plastique 7 destinée notamment à protéger le conducteur 5 et à délimiter les régions de contact cutanée 2 et de connexion 3. Au niveau de la région de contact cutané, la couche métallique mince 5 est revêtue d'une couche métallique plus épaisse 9 qui est striée. Les nervures ont par exemple une largeur de 1 µm et sont disposés à un pas de 1 µm. Toutefois, des valeurs comprises entre 0,1 et 5 µm pour la largeur et pour le pas pourront convenir. La hauteur de la couche métallique striée 9 est de l'ordre de 5 à 20 µm, de préférence de l'ordre de 10 à 15 µm.

Des expériences effectuées par la demanderesse ont montré qu'avec une électrode cutanée du type de celle représentée en figures 1A et 1B, dans laquelle les dimensions, en vue de dessus, de la région de contact cutané 2 sont de l'ordre de 3 à 5 mm de diamètre pour une structure circulaire, ou de 3 à 5 mm de côté pour une structure carrée, on obtient des signaux aussi nets et aussi intenses qu'avec une électrode plane classique d'un diamètre de l'ordre de 1 à 2 cm.

De l'avis de la demanderesse, bien que cela ne soit pas certain, cela s'explique par le fait que la partie supérieure de la peau, le stratum corneum, est relativement irrégulière et établit un mauvais contact avec une surface plane sauf si une force de pression intense est imposée. Par contre, avec une structure striée selon la présente invention, il y a une certaine pénétration du stratum corneum et donc un bien meilleur contact, la surface de contact étant augmentée de la surface interne des rainures formées dans l'électrode de contact. En outre, cette structure favorise le maintien de la sueur générée par les glandes sudoripares de la peau, la sueur conduisant à réduire la résistance de contact entre l'électrode et la peau.

Bien que les stries représentées en figure 1 aient une forme longitudinale, ceci ne constitue qu'un exemple destiné à faire comprendre l'invention. Toute forme de stries pourra être utilisée, par exemple un nervurage circulaire, ou encore, pour augmenter la surface du nervurage, une forme de fractales.

Un avantage de l'électrode cutanée représentée en figures 1A et 1B est que, étant donné sa structure et sa faible surface, la pression à appliquer pour la mettre en contact avec la peau est beaucoup plus faible que dans l'art antérieur. On peut donc utiliser des adhésifs moins puissants, moins susceptibles de léser l'épiderme du patient concerné.

Par ailleurs, on comprendra à partir de la description de la structure des figures 1A et 1B que la prise d'une connexion avec la partie de connexion 3 de l'électrode cutanée selon la présente invention est particulièrement simple. On pourra par exemple utiliser pour ce faire les enseignements du brevet américain 7090505 cédé à la demanderesse. En outre, la partie de liaison entre la connexion et la partie d'électrode cutanée proprement dite ayant une très faible épaisseur est très souple et ainsi un mouvement sur l'électrode de connexion n'est pas susceptible de décoller ou d'affecter d'une autre manière la partie de contact cutanée.

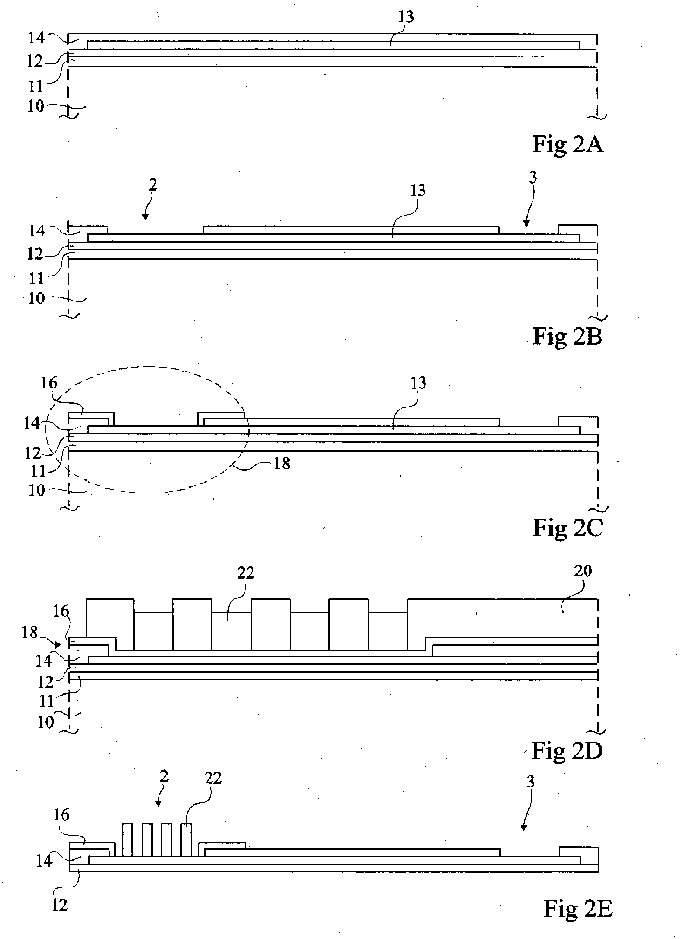

On va maintenant décrire en relation avec les figures 2A à 2E un procédé possible de réalisation d'une électrode cutanée telle que décrite précédemment.

Comme l'illustre la figure 2A, dans une première étape de fabrication, on dépose sur un support 10, par exemple une tranche de silicium, une couche mince d'un matériau 11 puis une couche d'une matière plastique 12, par exemple un polyimide.

La couche mince 11 doit être gravable sélectivement d'une part par rapport au support 10, d'autre part par rapport au matériau de la couche 12. Ainsi, dans une étape finale du procédé, on pourra détacher du support 10 la couche 12 et les éléments qui la recouvrent en éliminant la couche 11 par un procédé dit de lift off. Dans un exemple de réalisation, le support 10 est en silicium et la couche 11 est un oxyde de silicium.

La couche de matière plastique 12 peut être par exemple le polyimide commercialisé sous la référence PI 2611 par la société Dupont de Nemours. Ce matériau peut être déposé à la tournette sur une couche d'oxyde de silicium. La couche 12 pourra avoir après séchage une épaisseur de l'ordre de 3 à 5 µm pour assurer ensuite la tenue mécanique de la structure obtenue.

Sur la couche 12, qui correspond au support 1 de la figure 1A, on dépose une couche de matériau conducteur 13 que l'on grave pour lui donner la forme souhaitée de la couche 5 telle que représentée en figure 1A. La couche 13 peut par exemple être une couche d'or d'une épaisseur inférieure à 1 µm. Tout autre matériau métallique pourra être adopté par l'homme de l'art, du moment qu'il présente une tenue suffisante, qu'il résiste à une torsion, et qu'il peut être déposé selon une faible épaisseur de l'ordre de 0,5 à 3 µm.

Sur la couche 13, on dépose une nouvelle couche de matière plastique 14, par exemple à nouveau une couche de polyimide. La couche 14 correspond à la couche 7 de la figure 1A.

A l'étape illustrée en figure 2B, on a ouvert deux fenêtres 2 et 3 dans la couche de matière plastique 14. Divers procédés connus pourront être utilisés pour ouvrir ces fenêtres. Par exemple, si le matériau constituant la couche 14 est un polyimide difficilement gravable sélectivement par rapport à une résine photosensible, on commence par déposer une couche d'aluminium ou autre, destinée à former un masque dur, puis on grave les ouvertures souhaitées correspondant aux régions 2 et 3 dans la couche d'aluminium et on se sert ensuite de la couche d'aluminium comme masque pour graver dans la couche de polyimide la fenêtre 2 correspondant au contact cutané et la fenêtre 3 correspondant à la zone de connexion.

A l'étape illustrée en figure 2C, on a déposé une couche conductrice mince 16 dans l'ouverture 2 et autour de celle-ci. Cette couche conductrice est destinée à permettre d'établir un contact lors d'une étape ultérieure de dépôt électrolytique.

La figure 2D représente de façon agrandie la partie entourée du cercle en pointillés 18 en figure 2C. On a déposé sur la structure un masque, par exemple un masque de résine 20, dans lequel on a formé des ouvertures. Dans ces ouvertures, on a déposé par dépôt électrolytique un métal 22 tel que du cuivre. Si le dépôt se fait autrement que par voie électrolytique, il n'est pas nécessaire de prévoir la couche conductrice mince 16 décrite précédemment.

Enfin, à l'étape illustrée en figure 2E, on a éliminé la couche de résine 20, et séparé un dispositif selon l'invention du support 10 en éliminant la couche de lift off 11 pour détacher la structure comprenant la couche de polyimide 12 et les éléments qui la revêtent. La structure illustrée en figure 2E correspond à celle illustrée en figures 1A et 1B.

Le procédé décrit ci-dessus ne constitue qu'un exemple considéré actuellement comme avantageux de réalisation de l'invention mais de nombreux autres procédés de réalisation pourront être adoptés. Egalement, d'autres matériaux que du polyimide, de l'or et du cuivre pourront être utilisés du moment que chacun de ces matériaux, et notamment ceux remplaçant le polyimide et l'or assurent le rôle de souplesse qui leur est imparti ainsi que le rôle de tenue mécanique sur une très faible épaisseur.

D'autres variantes de la présente invention pourront être réalisées. Par exemple, si on considère la vue de dessus de la figure 1B, au lieu d'avoir une électrode ronde ou carrée, on pourra prévoir que cette électrode est en étoile ou a toute autre forme souhaitée, de façon par exemple à faciliter sa mise en place à certains emplacements souhaités, par exemple dans la chevelure, en évitant d'avoir à couper une trop grande surface de cheveux, les doigts de l'étoile se glissant entre les mèches de cheveux. On pourra aussi prévoir que la zone de contact comporte une ouverture centrale.

Alors que l'on a décrit une réalisation particulière et avantageuse de l'invention, on comprendra que l'invention prévoit de façon générale, une électrode cutanée comprenant, sur un support souple, un plot de contact avec la peau d'une faible épaisseur muni de stries d'une profondeur supérieure à 10 µm, et d'un pas et d'une largeur de 0,1 à 5 µm, ce plot étant connecté à une zone de prise de connexion. La liaison électrique entre le plot et la zone de prise de connexion peut se faire par tout moyen, et pas nécessairement par une sous-couche conductrice. Le plot n'est pas nécessairement en métal mais doit seulement avoir une surface conductrice, y compris dans les stries.

En ce qui concerne le procédé de fabrication toute technique connue pourra être utilisée pour former un plot strié, par exemple des méthodes d'électro-érosion. Un bloc de cuivre strié pourra être rapporté sur un support souple.

Par ailleurs, on a décrit une application particulière de l'électrode pour détecter des signaux électriques sur la peau d'un patient. On pourra aussi prévoir que cette électrode est disposée à l'intérieur du corps, par exemple sur le muscle cardiaque, autour d'un nerf, etc.

## Revendications

1. Electrode cutanée comprenant, sur un support souple (1) d'une épaisseur inférieure à 10 µm, un plot (9) d'une épaisseur inférieure à 30 µm muni de stries d'une profondeur supérieure à 10 µm, les surfaces au moins de ce plot étant conductrices et étant connectées par un conducteur unique (5) à une zone de prise de connexion (3).

2. Electrode cutanée selon la revendication 1, comprenant :
une première couche isolante support (1) d'une épaisseur inférieure à 10 µm ;
une couche métallique (5-9) d'une épaisseur inférieure à 20 µm munie de stries d'une profondeur supérieure à 10 µm au niveau d'une zone de contact ; et
une deuxième couche isolante (7) d'une épaisseur inférieure à 10 µm revêtant la structure en dehors des zones de contact (2) et d'une zone de prise de connexion (3).

3. Electrode cutanée selon la revendication 2, dans laquelle les première et deuxième couches isolantes sont des couches de polyimide d'une épaisseur de l'ordre de 2 à 3 µm.

4. Electrode cutanée selon la revendication 2 ou 3, dans laquelle la couche métallique comprend :
une première couche métallique (5) d'une épaisseur inférieure à 5 µm ; et
une deuxième couche métallique (9) formée sur la première couche métallique à l'emplacement de la zone de contact cutané, d'une épaisseur supérieure à 5 µm et nervurée sur toute son épaisseur, les nervures étant ménagées à un pas de l'ordre de 0,5 à 3 µm.

5. Electrode cutanée selon l'une quelconque des revendications 2 à 4, comprenant, sur la première couche isolante, une extension de la couche métallique jusqu'à une zone de connexion (3).

6. Electrode cutanée selon l'une quelconque des revendications 1 à 5, ayant une surface de l'ordre de 0,1 cm².

7. Procédé de fabrication d'une électrode cutanée comprenant :
former sur un support (10) une couche sélectivement gravable (11) ;
former une première couche de matière plastique (12) ;
former une première couche métallique (13).;
graver la première couche métallique (13) pour lui donner la forme d'une couche qui définit un conducteur (5) unique de l'électrode cutanée ;
former une deuxième couche de matière plastique (14) ;
ouvrir dans la deuxième couche en matière plastique au-dessus de la première couche métallique (13) des ouvertures (2) et (3) dans des régions respectives de contact et de connexion ;
former sur la première couche métallique (13), dans la région de contact, des surépaisseurs métalliques nervurées d'une hauteur de 5 à 20 µm, à un pas de l'ordre de 1 à 5 µm ; et
séparer du substrat l'ensemble de la première couche isolante (12) et des éléments qu'elle supporte, par élimination de la couche sélectivement gravable (11).

8. Procédé selon la revendication 7, dans lequel le support (10) est une plaquette de silicium et la couche sélectivement gravable (11) est une couche d'oxyde de silicium.

9. Procédé selon la revendication 7 ou 8, dans lequel les couches de matière plastique sont des couches de polyimide d'une épaisseur de 2 à 10 µm, de préférence de 3 à 5 µm.

10. Procédé selon l'une quelconque des revendications 7 à 9, dans lequel la première couche métallique (13) est une couche d'or d'une épaisseur d'environ 1 µm.

## Patentansprüche

1. Eine Hautelektrode, wobei die Hautelektrode Folgendes aufweist: auf einem flexiblen Träger (1) mit einer Dicke, die kleiner als 10 µm ist, ein Pad (9) bzw. Kissen mit einer Dicke, die kleiner als 30µm ist und das Nuten aufweist, mit einer Tiefe, die größer als 10 µm sind, wobei die Oberflächen von wenigstens dem Pad leitend sind und durch einen einzelnen Leiter (5) mit einem Verbindungsbereich (3) verbunden sind.

2. Die Hautelektrode nach Anspruch 1, wobei die Hautelektrode ferner Folgendes aufweist:
eine erste isolierende Unterstützungselektrode (1) mit einer Dicke, die kleiner ist als 10 µm;
eine Metallschicht (5-9) mit einer Dicke, die kleiner ist als 20 µm und die Nuten aufweist mit einer Tiefe, die größer ist als 10 µm im Bereich des Verbindungsbereichs; und
eine zweite isolierende Schicht (7) mit einer Dicke, die geringer ist als 10 µm und die die Struktur außerhalb von Verbindungsbereichen (2) und eines Verbindungsbereichs (3) ummantelt.

3. Die Hautelektrode nach Anspruch 2, wobei die erste und die zweite Isolierschicht Polyimidschichten sind mit einer Dicke, die im Bereich von 2 bis 3 µm liegt.

4. Die Hautelektrode nach Anspruch 2 oder 3, wobei die Metallschicht Folgendes aufweist:
eine erste Metallschicht (5) mit einer Dicke, die kleiner ist als 5 µm; und
eine zweite Metallschicht (9), die auf der ersten Metallschicht, im Bereich der Hautkontaktfläche, ausgebildet ist mit einer Dicke, die größer ist als 5 µm und die über ihre gesamte Dicke mit Nuten versehen ist, wobei die Nuten mit einem Abstand im Bereich von 0,5 bis 3 µm ausgebildet sind.

5. Die Hautelektrode nach einem der Ansprüche 2 bis 4, wobei die Hautelektrode ferner Folgendes aufweist:
eine Erstreckung der Metallschicht bis zu dem Verbindungsbereich (3) auf der ersten Isolierschicht.

6. Die Hautelektrode nach einem der Ansprüche 1 bis 5, wobei die Hautelektrode eine Oberfläche in der Größenordnung von 0,1 cm² aufweist.

7. Ein Hautelektroden-Herstellungsverfahren, wobei das Verfahren folgende Schritte aufweist:
Ausbilden auf einem Träger (10) einer wahlweise ätzbaren Schicht (11);
Ausbilden einer ersten Schicht aus Plastikmaterial (12);
Ausbilden einer ersten Metallschicht (13);
Ätzen der ersten Metallschicht (13) um der Metallschicht die Form einer Schicht zu geben, die einen ersten Leiter (5) der Hautelektrode definiert;
Ausbilden einer zweiten Schicht von Plastikmaterial (14);
Öffnen, in der zweiten Schicht von Plastikmaterial über der ersten Metallschicht (13), von Öffnungen (2) und (3) in entsprechenden Kontakt- und Verbindungsbereichen;
Ausbilden auf der ersten Metallschicht (13), in dem Kontaktbereich der mit Nuten versehen ist, von erhabenen Metallbereichen mit einer Höhe von 5 bis 20 µm und mit einem Abstand im Bereich von 1 bis 5 µm; und
Separieren von dem Substrat, die Anordnung der ersten Isolierschicht (12) und der Elemente die sie trägt, durch Entfernen der wahlweise ätzbaren Schicht (11).

8. Das Verfahren nach Anspruch 7, wobei der Träger (10) ein Siliziumwafer ist und wobei die wahlweise ätzbare Schicht (11) eine Siliziumoxidschicht ist.

9. Das Verfahren nach einem der Ansprüche 7 oder 8, wobei die Schichten aus Plastikmaterial Polyimidschichten sind mit einer Dicke im Bereich von 2 bis 10 µm, vorzugsweise im Bereich von 3 bis 5 µm.

10. Das Verfahren nach einem der Ansprüche 7 bis 9, wobei die erste Metallschicht (13) eine Goldschicht ist, mit einer Dicke von ungefähr 1 µm.

## Claims

1. A cutaneous electrode comprising, on a flexible support (1) having a thickness smaller than 10 µm, a pad (9) having a thickness smaller than 30 µm provided with grooves having a depth greater than 10 µm, the surfaces at least of this pad being conductive and being connected by a single conductor (5) to a connection area (3).

2. The cutaneous electrode of claim 1, comprising:
a first insulating support electrode (1) having a thickness smaller than 10 µm;
a metal layer (5-9) having a thickness smaller than 20 µm provided with grooves having a depth greater than 10 µm at the level of a contacting area; and
a second insulating layer (7) having a thickness smaller than 10 µm coating the structure outside of the contacting areas (2) and of a connection area (3).

3. The cutaneous electrode of claim 2, wherein the first and second insulating layers are polyimide layers having a thickness in the range from 2 to 3 µm.

4. The cutaneous electrode of claim 2 or 3, wherein the metal layer comprises:
a first metal layer (5) having a thickness smaller than 5 µm; and
a second metal layer (9) formed on the first metal layer at the location of the cutaneous contact area, having a thickness greater than 5 µm and grooved across its entire thickness, the grooves being formed with a pitch in the range from 0.5 to 3 µm.

5. The cutaneous electrode of any of claims 2 to 4, comprising, on the first insulating layer, an extension of the metal layer all the way to a connection area (3).

6. The cutaneous electrode of any of claims 1 to 5, having a surface area in the order of 0.1 cm².

7. A cutaneous electrode manufacturing method, comprising:
forming on a support (10) a selectively-etchable layer (11);
forming a first layer of plastic material (12);
forming a first metal layer (13);
etching the first metal layer (13) to give it the shape of a layer which defines a single conductor (5) of the cutaneous electrode;
forming a second layer of plastic material (14);
opening in the second layer of plastic material above the first metal layer (13) openings (2) and (3) in respective contact and connection areas;
forming on the first metal layer (13), in the contact region, grooved metal raised areas having a height from 5 to 20 µm, with a pitch in the range from 1 to 5 µm; and
separating from the substrate the assembly of the first insulating layer (12) and of the elements that it supports, by removing the selectively-etchable layer (11).

8. The method of claim 7, wherein the support (10) is a silicon wafer and the selectively-etchable layer (11) is a silicon oxide layer.

9. The method of claim 7 or 8, wherein the layers of plastic material are polyimide layers having a thickness in the range from 2 to 10 µm, preferably from 3 to 5 µm.

10. The method of any of claims 7 to 9, wherein the first metal layer (13) is a gold layer having a thickness of approximately 1 µm.
